(19) 

**Europäisches Patentamt**

**European Patent Office**

**Office européen des brevets**

(11) **EP 4 289 379 A1**

(12) **EUROPEAN PATENT APPLICATION**
published in accordance with Art. 153(4) EPC

(43) Date of publication:
**13.12.2023 Bulletin 2023/50**

(21) Application number: **21924032.2**

(22) Date of filing: **14.05.2021**

(51) International Patent Classification (IPC):
***A61B 34/10*** (2016.01)          ***A61B 34/30*** (2016.01)

(86) International application number:
**PCT/CN2021/093787**

(87) International publication number:
**WO 2022/166024 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.02.2021   CN 202110143688**

(71) Applicant: **Harbin Intelligent Surgery Equipment Co., Ltd.**
**Harbin, Heilongjiang 150000 (CN)**

(72) Inventor: **WANG, Wei**
**Harbin, Heilongjiang 150000 (CN)**

(74) Representative: **Zaboliene, Reda**
**Metida**
**Business center Vertas**
**Gyneju str. 16**
**01109 Vilnius (LT)**

(54) **METHOD AND DEVICE FOR PLANNING INITIAL POSES OF SURGICAL ARMS OF LAPAROSCOPIC SURGICAL ROBOT**

(57)     The present invention discloses a method and a device for planning initial poses of surgical arms of a laparoscopic surgical robot, comprising: acquiring positions set for a minimally invasive incision and a surgical region; obtaining a plurality of initial position schemes for a surgical arm joint on the basis of the position of the minimally invasive incision and the position of the surgical region; and calculating performance indicators of each initial position scheme for the surgical arm joint according to the position of the surgical region respectively. In the present invention, a plurality of initial position schemes for the surgical arm joint are obtained on the basis of the position of the minimally invasive incision and the position of the surgical region, and performance indicators of each initial position scheme for the surgical arm joint are calculated. Medical staff can intuitively analyze the advantages and disadvantages of a plurality of surgical arm joint initial position schemes by observing the performance indicators. Therefore, on the one hand, this method can reduce the workload of medical staff. On the other hand, it can quickly complete the preoperative preparation of robot-assisted minimally invasive surgery, and increase the speed of medical staff setting the initial poses of surgical arm.

Fig. 1

**Description**

**Technical Field**

The present invention relates to the technical field of laparoscopic surgery and, in particular, to a method and a device for planning initial poses of surgical arms of a laparoscopic surgical robot.

**Background Art**

Unlike the traditional minimally invasive surgery, when the laparoscopic surgical robot is used to perform the minimally invasive surgery, the initial poses of the surgical arms should be adjusted at the same time of selecting the minimally invasive incision position. The movement performance of the surgical arms and the interference between the surgical arms should be considered. The unreasonable initial poses of the surgical arms may lead to insufficient working space of the micro-instruments in the body, which causes difficult reaching to the target area and even the collision of the surgical arm. Thus, the surgery cannot be continued.

Based on the existing laparoscopic surgical robots, since the medical staff does not know the robotics related knowledge, the preoperative preparation of robot-assisted minimally invasive surgery can only rely on long-term clinical experience to make subjective judgments. It cannot quickly complete the preoperative planning preparation for the surgical arm.

**Summary of the Invention**

The problem addressed by the present invention is how to quickly complete preoperative planning preparation for surgical arms.

In order to solve the above problems, the present invention provides a method for planning initial poses of surgical arms of a laparoscopic surgical robot, comprising:

acquiring positions set for a minimally invasive incision and a surgical region;
obtaining a plurality of initial position schemes for a surgical arm joint on the basis of the position of the minimally invasive incision and the position of the surgical region; and
calculating performance indicators of each initial position scheme for the surgical arm joint according to the position of the surgical region respectively.

Optionally, the performance indicators include micro-instrument tip dexterity, micro-instrument intra-body cooperation space, and hand-eye coordination.

Optionally, the micro-instrument tip dexterity is used to reflect conversion capability of the speed and force of the surgical arm from a joint space to a tip task space, and the micro-instrument tip dexterity is obtained by the following formula:

$$\text{Micro-instrument tip dexterity} = \begin{cases} \dfrac{\int_w \dfrac{\mu_\sigma}{\sigma_\sigma} \mathrm{d}w}{\int_w \mathrm{d}w}, & \sigma_{G\min} \neq 0 \\ 0, & \sigma_{G\min} = 0 \end{cases}$$

where $\mu_\sigma$ and $\sigma_\sigma$ are a mean value and a standard deviation of singular values of a Jacobian matrix corresponding to a tip posture of the micro-instrument respectively;

$\sigma_{G\min}$ is a minimum singular value of the Jacobian matrix in the surgical region;

w is a minimally invasive surgical region space.

Optionally, the greater the value of the micro-instrument tip dexterity is, the more balanced the conversion capability of the speed and force of the surgical arm from the joint space to the tip task space is.

Optionally, the micro-instrument intra-body cooperation space is used to reflect a cooperative space for the left and right micro-instruments at the position of the surgical region on the basis of a collision-free working space for the left and right surgical arms at the position of the surgical region, and the micro-instrument intra-body cooperation space is obtained by the following formula:

$$\text{Micro} - \text{instrument intra} - \text{body cooperation space} = \frac{v_{cr} \cap v_{cc}}{v_c} \times 100\%, v_{cr} \in v_c, v_{cc} \in v_c$$

where $v_c$ is the surgical region position space;

$v_{cr}$ is the cooperative space for the left and right micro-instruments at the position of the surgical region;

$v_{cc}$ is a collision-free working space for the left and right surgical arms at the position of the surgical region.

Optionally, the greater the value of the micro-instrument intra-body cooperation space is, the larger the cooperative space for the left and right micro-instruments at the position of the surgical region is on the basis of the collision-free working space for the left and right surgical arms at the position of the surgical region.

Optionally, the hand-eye coordination is used to reflect a positional relationship between the left and right micro-instruments and a visual field relationship between the left and right micro-instruments and an endoscope; the positional relationship between the left and right micro-instruments includes an operating angle of the left and right micro-instruments and an elevation angle of the left and right micro-instruments; and the visual field relationship between the left and right micro-instruments and the endoscope includes an azimuth angle of the left and right micro-instruments relative to the direction of visual field.

Optionally, the operating angle of the left and right micro-instruments is 60°; the elevation angle of the left and right micro-instruments is 60°; and the azimuth angles of the left and right micro-instruments relative to the direction of visual field are the same.

Optionally, the obtaining a plurality of initial position schemes for a surgical arm joint on the basis of the position of the minimally invasive incision comprises:

performing a kinematic inverse operation according to the position of the minimally invasive incision and the position of the surgical region to obtain a plurality of combinations of the positions of the surgical arm joint;

performing an inter-arm collision test on the plurality of the surgical arm joint position combinations to obtain a plurality of initial position schemes for the surgical arm joint that do not generate an inter-arm collision.

The present invention also provides a device for planning initial poses of surgical arms of a laparoscopic surgical robot, comprising:

an acquisition module configured for acquiring positions set for a minimally invasive incision and a surgical region;

a scheme generation module configured for obtaining a plurality of initial position schemes for a surgical arm joint on the basis of the position of the minimally invasive incision and the position of the surgical region; and

a calculation module configured for calculating performance indicators of each initial position scheme for the surgical arm joint respectively.

Advantageous effects of the invention. In the present invention, a plurality of initial position schemes for the surgical arm joint are obtained on the basis of the position of the minimally invasive incision and the position of the surgical region, and the performance indicators of each initial position scheme for the surgical arm joint are respectively calculated. The medical staff can intuitively analyze the advantages and disadvantages of the plurality of surgical arm joint initial position schemes by observing the performance indicators, without knowing the robot structure and the calculation process of the corresponding kinematics. Therefore, on the one hand, this method can reduce the workload of medical staff. On the other hand, it can quickly complete the preoperative preparation of robot-assisted minimally invasive surgery, and increase the speed of medical staff setting the initial poses of surgical arm. At the same time, the medical staff can select an appropriate initial position scheme for the surgical arm joint to adjust the initial poses of the surgical arms according to needs, so as to ensure the operating performance of the surgical robot. Therefore, the overall performance of the surgical robot can be better used during the surgery, and the working efficiency of the surgical arms during the surgery can be enhanced.

**Brief Description of the Drawings**

Fig. 1 is a flowchart of a method for planning initial poses of surgical arms of a laparoscopic surgical robot according to an embodiment of the present invention;

Fig. 2 is a structure diagram showing a positional relationship between left and right micro-instruments and a visual field relationship between the left and right micro-instruments and an endoscope according to the embodiment of the present invention;

Fig. 3 is a flowchart for obtaining a plurality of initial position schemes for a surgical arm joint on the basis of a position of a minimally invasive incision and a position of a surgical region according to an embodiment of the present invention;

Fig. 4 is a diagram of the operation of setting the position of the minimally invasive incision and the position of the surgical region on the basis of a preoperative image according to an embodiment of the present invention;

Fig. 5 is a structure diagram of a device for planning initial poses of surgical arms of a laparoscopic surgical robot according to an embodiment of the present invention.

**Detailed Description of the Invention**

To make the above objects, features and advantages of the present invention more apparent, a detailed description of specific embodiments of the present invention will be made with reference to the accompanying drawings.

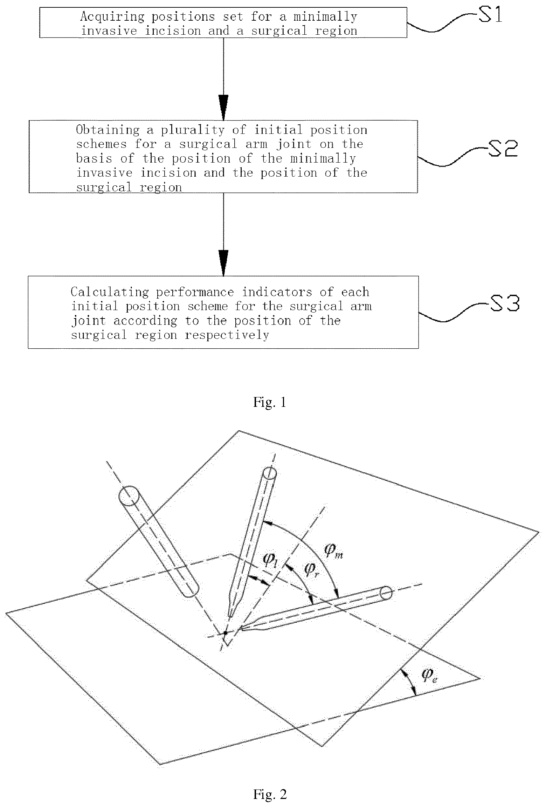

As shown in Fig. 1, a method for planning initial poses of surgical arms of a laparoscopic surgical robot according to an embodiment of the present invention includes:

Step S1, acquiring positions set for a minimally invasive incision and a surgical region;

Step S2, obtaining a plurality of initial position schemes for a surgical arm joint on the basis of the position of the minimally invasive incision and the position of the surgical region; and

Step S3, calculating performance indicators of each initial position scheme for the surgical arm joint according to the position of the surgical region respectively.

In this embodiment, the control system acquires positions set for a minimally invasive incision and a surgical region. Herein, the position of the minimally invasive incision and the position of the surgical region may be set by a medical staff via a human-computer interaction interface, and the medical staff may fine-tune an intervention position of the surgical arm. Thereafter, the control system calculates the position of the minimally invasive incision to obtain a plurality of initial position schemes for the surgical arm joint (i.e., initial poses of the surgical arms or a form in which the surgical arms are connected with the patient). Then, the control system calculates performance indicators of each initial position scheme for the surgical arm joint according to the position of the surgical region, so that the medical staff can select a suitable initial position scheme for the surgical arm joint according to the performance indicators.

The medical staff selects the initial position scheme for the surgical arm joint corresponding to the performance indicators according to the requirements, and adjusts the initial poses of the surgical arm joint according to the initial position scheme for the surgical arm joint so as to complete the preoperative positioning preparation process of the surgical robot.

In summary, the control system obtains a plurality of initial position schemes for the surgical arm joint on the basis of the position of the minimally invasive incision and the position of the surgical region, and calculates the performance indicators of each initial position scheme for the surgical arm joint respectively. The medical staff can intuitively analyze the advantages and disadvantages of the plurality of surgical arm joint initial position schemes by observing the performance indicators, without knowing the robot structure and the calculation process of the corresponding kinematics. Therefore, on the one hand, this method can reduce the workload of medical staff. On the other hand, it can quickly complete the preoperative preparation of robot-assisted minimally invasive surgery, and increase the speed of medical staff setting the initial poses of surgical arm. At the same time, the medical staff can select an appropriate initial position scheme for the surgical arm joint to adjust the initial poses of the surgical arms according to needs, so as to ensure the operating performance of the surgical robot. Therefore, the overall performance of the surgical robot can be better used during the surgery, and the working efficiency of the surgical arms during the surgery can be enhanced.

Optionally, the performance indicators include micro-instrument tip dexterity, micro-instrument intra-body cooperation space, and hand-eye coordination.

In this embodiment, the micro-instrument tip dexterity, the micro-instrument intra-body cooperation space, and the hand-eye coordination for each initial position of the surgical arm joint are calculated respectively from the surgical region position. By converting the performance indicators into the micro-instrument tip dexterity, the micro-instrument intra-body cooperation space, and the hand-eye coordination, the performance indicators are quantified to facilitate the calculation of the control system. Meanwhile, after quantification, the medical staff can intuitively determine the advantages and disadvantages of the performance indicators, so as to facilitate the medical staff to select an appropriate initial position scheme for the surgical arm joint according to the performance indicators.

Optionally, the micro-instrument tip dexterity is used to reflect conversion capability of the speed and force of the surgical arm from a joint space to a tip task space, and the micro-instrument tip dexterity is obtained by the following formula:

$$
\text{Micro-instrument tip dexterity} = \begin{cases} \dfrac{\int_w \dfrac{\mu_\sigma}{\sigma_\sigma}\,dw}{\int_w dw}, & \sigma_{G\min} \neq 0 \\ 0, & \sigma_{G\min} = 0 \end{cases}
$$

where $\mu_\sigma$ and $\sigma_\sigma$ are a mean value and a standard deviation of singular values of a Jacobian matrix corresponding to a tip posture of the micro-instrument respectively;

$\sigma_{G\min}$ is a minimum singular value of the Jacobian matrix in the surgical region;

w is a minimally invasive surgical region space.

In the present embodiment, the joint space refers to a space in which a link of the surgical arm is freely movable, and the tip task space refers to a space in which the tip of the micro-instrument moves and rotates in the X, Y and Z axis directions. Therefore, the performance of the surgical robot with different initial position schemes for the surgical arm joint can be judged according to the dexterity of the tip of the micro-instrument, and a reasonable initial position scheme for the surgical arm joint can be selected.

Optionally, the greater the value of the micro-instrument tip dexterity is, the more balanced the conversion capability of the speed and force of the surgical arm from the joint space to the tip task space is.

In this embodiment, when selecting a suitable initial position scheme for the surgical arm joint from a plurality of initial position schemes for the surgical arm joint, it is necessary to maximize the dexterity of the tip of the micro-instrument if it is to ensure that the speed and force conversion capability of the surgical arm from the joint space to the tip task space is balanced. Therefore, the medical staff can select an initial position scheme for the surgical arm joint corresponding to the maximum dexterity of the micro-instrument tip, so as to ensure that the speed and force conversion capability of the surgical arm from the joint space to the tip task space is balanced.

Optionally, the micro-instrument intra-body cooperation space is used to reflect a cooperative space for the left and right micro-instruments at the position of the surgical region on the basis of a collision-free working space for the left and right surgical arms at the position of the surgical region, and the micro-instrument intra-body cooperation space is obtained by the following formula:

$$
\text{Micro} - \text{instrument intra} - \text{body cooperation space} = \frac{v_{cr} \cap v_{cc}}{v_c} \times 100\%, v_{cr} \in v_c, v_{cc} \in v_c
$$

where $v_c$ is the surgical region position space;
$v_{cr}$ is the cooperative space for the left and right micro-instruments at the position of the surgical region;
$v_{cc}$ is a collision-free working space for the left and right surgical arms at the position of the surgical region.

In this embodiment, since the left and right surgical arms are disposed outside the body, the left and right micro-instruments are disposed inside the body. When the left and right surgical arms collide outside the body, the left and right micro-instruments cannot cooperate in the body. Therefore, it is necessary to eliminate the situation that the left and right surgical arms collide outside the body. In the formula, by taking the intersection of the cooperative space of the left and right micro-instruments at the position of the surgical region and the collision-free working space of the left and right surgical arms at the position of the surgical region, the cooperative space of the left and right micro-instruments at the position of the surgical region can be obtained on the basis of the collision-free working space of the left and right surgical arms at the position of the surgical region.

Optionally, the greater the value of the micro-instrument intra-body cooperation space is, the larger the cooperative space for the left and right micro-instruments at the position of the surgical region is on the basis of the collision-free working space for the left and right surgical arms at the position of the surgical region.

In this embodiment, in order to better ensure the progress of the surgical process during the surgery, it is necessary to ensure that the left and right micro-instruments have sufficient cooperative space in the surgical region. It is generally necessary to maximize the cooperative space of the left and right micro-instruments in the surgical region. Thus, the medical staff can select an initial position scheme for the surgical arm joint corresponding to the maximum the micro-instrument intra-body cooperation space, so that the cooperative space of the left and right micro-instruments in the surgical region can be maximized on the basis of the collision-free working space of the left and right surgical arms at the position of the surgical region.

Optionally, as shown in Fig. 2, the hand-eye coordination of the present embodiment is used to reflect a positional relationship between the left and right micro-instruments and a visual field relationship between the left and right micro-instruments and the endoscope. The positional relationship between the left and right micro-instruments includes an operating angle $\varphi_m$ of the left and right micro-instruments. The left micro-instrument and the right micro-instrument are located on an instrument plane (namely, an inclined plane of Fig. 2). $\varphi_m$ is an included angle between the left instrument and the right instrument. The elevation angle of the left and right micro-instruments is $\varphi_e$. Specifically, $\varphi_e$ is an included angle between the instrument plane and a fitting operation plane (i.e., the plane of Fig. 2). The visual field relationship between the left and right micro-instruments and the endoscope includes the azimuth angle $\varphi_l$, $\varphi_r$ of the left and right micro-instruments with respect to the direction of the visual field. Specifically, $\varphi_l$, $\varphi_r$ are an included angle between the left micro-instrument and the projection of the optical axis and an included angle between the right micro-instrument and the projection of the optical axis. The endoscope illuminates the plane of the instrument to form an optical axis of the endoscope between the endoscope and the plane of the instrument, and the projection of the optical axis of the endoscope on the plane of the instrument is the projection of the optical axis.

In this embodiment, by calculating and obtaining the operating angle of the left and right micro-instruments, the elevation angle of the left and right micro-instruments and the azimuth angles of the left and right micro-instruments relative to the direction of visual field respectively, the positional relationship between the left and right micro-instruments and the visual field relationship between the left and right micro-instruments and the endoscope can be quantified, thereby facilitating the medical staff to determine the hand-eye coordination according to the operating angle of the left and right micro-instruments, the elevation angle of the left and right micro-instruments and the azimuth angles of the left and right micro-instruments relative to the direction of visual field.

Optionally, the operating angle of the left and right micro-instruments is $\varphi_m$ =60°; the elevation angle of the left and right micro-instruments is $\varphi_e$ =60°; and the azimuth angles of the left and right micro-instruments relative to the direction of visual field are $\varphi_1 = \varphi_r$.

In this embodiment, based on the calculation and verification, it can be determined that the hand-eye coordination is the best when the operating angle of the left and right micro-instruments is $\varphi_m$ =60°, the elevation angle of the left and right micro-instruments is $\varphi_e$ =60°, and the azimuth angle of the left and right micro-instruments relative to the direction of visual field is $\varphi_1 = \varphi_r$. From this, it can be determined that the hand-eye coordination is better when the operating angle of the left and right micro-instruments is closer to 60°, the elevation angle of the left and right micro-instruments is closer to 60°, and the azimuth angles of the left and right micro-instruments relative to the direction of visual field are closer to the same. Therefore, when selecting a specific initial position scheme for an surgical arm joint, a planning scheme with better hand-eye coordination can be selected as the initial position scheme for the surgical arm joint when the operating angle of the left and right micro-instruments is close to 60°, the elevation angle of the left and right micro-instruments is close to 60°, and the azimuth angles of the left and right micro-instruments relative to the direction of visual field are approximately the same.

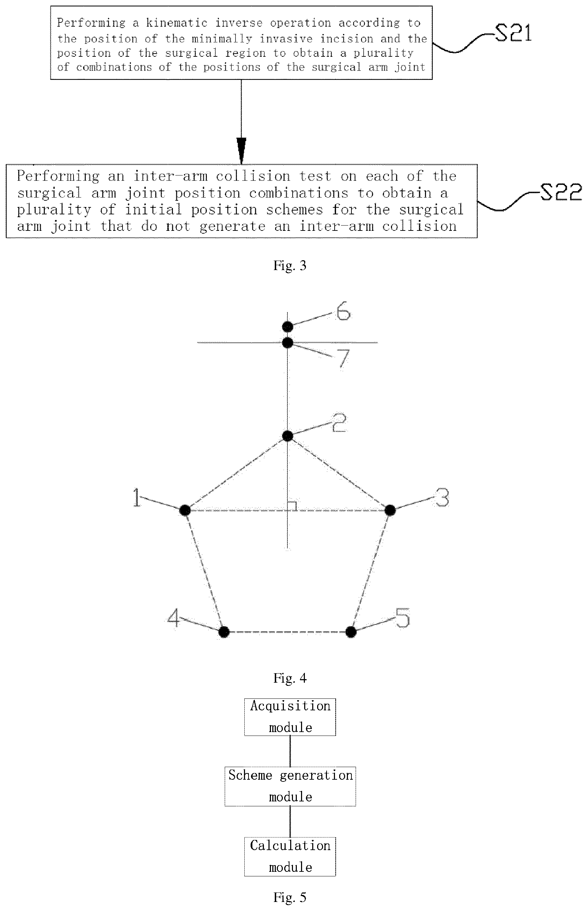

Optionally, as shown in Fig. 3, the obtaining a plurality of initial position schemes for a surgical arm joint based on the position of the minimally invasive incision includes:

S21, performing a kinematic inverse operation according to the position of the minimally invasive incision to obtain a plurality of combinations of the positions of the surgical arm joint;
S22, performing an inter-arm collision test on the plurality of the surgical arm joint position combinations to obtain a plurality of initial position schemes for the surgical arm joint that do not generate an inter-arm collision.

In this embodiment, the unreasonable initial poses of the surgical arms may cause collision between the surgical arms, so that the surgery cannot be continued. By performing an inter-arm collision test on a plurality of surgical arm joint position combinations, a plurality of initial position schemes for the surgical arm joint which do not generate an inter-arm collision can be obtained, so that it can be ensured that the initial position scheme for the surgical arm joint selected by the medical staff does not generate collision, and thus it can be ensured that the surgery can be continuously and orderly performed.

Optionally, the position of the minimally invasive incision and the position of the surgical region are set in a manner of setting the position of the minimally invasive incision and the position of the surgical region by using a cross positioning laser and according to the preoperative image.

In the present embodiment, as shown in Fig. 4, taking the minimally invasive incision setting of lower abdominal surgery in the urinary surgery as an example, a left instrument arm intervention position 1, an endoscope-holding arm intervention position 2 and a right instrument arm intervention position 3 are positioned in an isosceles triangle. A connection line between a center 6 of the surgical region position and the endoscope-holding arm intervention position 2 is perpendicular to a connection line between the left instrument arm intervention position 1 and the right instrument arm intervention position 3. Herein, the position parameters of different surgical methods and patient body types will be slightly different. The corresponding parameter settings can be adjusted by the software interface, and the abut positioning can be established with the patient by the cross positioning laser of the robotic system. The cross lines are respectively parallel to the connection line of the left instrument arm intervention position 1 and the right instrument arm intervention position 3 and coincide with the connection line of the endoscope-holding arm intervention position 2 and the center 6 of the surgical region position. Herein, the position of the minimally invasive incision further includes two ancillary instrument intervention positions 4 and 5, and a cross positioning laser center position 7.

As shown in Fig. 5, a device for planning initial poses of surgical arms of a laparoscopic surgical robot according to another embodiment of the present invention includes:

an acquisition module configured for acquiring positions set for a minimally invasive incision and a surgical region; a scheme generation module configured for obtaining a plurality of initial position schemes for a surgical arm joint on the basis of the position of the minimally invasive incision and the position of the surgical region; and a calculation module configured for calculating performance indicators of each initial position scheme for the surgical arm joint according to the position of the surgical region respectively.

In this embodiment, the acquisition module acquires positions of a minimally invasive incision and a surgical region set by the medical staff. Then, the scheme generation module calculates the position of the minimally invasive incision to obtain a plurality of initial position schemes for the surgical arm joint (i.e., initial poses of the surgical arms or a form in which the surgical arms abut with the patient). Then, the calculation module calculates performance indicators of each initial position scheme for the surgical arm joint according to the position of the surgical region respectively, so that the medical staff can select a suitable initial position scheme for the surgical arm joint according to the performance indicators.

Although the present disclosure has been described above, the scope of protection of the present disclosure is not limited thereto. Various changes and modifications may be made by those skilled in the art without departing from the spirit and scope of this disclosure, and such changes and modifications are intended to fall within the scope of this disclosure.

**Claims**

1. A method for planning initial poses of surgical arms of a laparoscopic surgical robot, comprising:

   acquiring positions set for a minimally invasive incision and a surgical region;
   obtaining a plurality of initial position schemes for a surgical arm joint on the basis of the position of the minimally invasive incision and the position of the surgical region; and
   calculating performance indicators of each initial position scheme for the surgical arm joint according to the position of the surgical region respectively.

2. The method for planning initial poses of surgical arms of a laparoscopic surgical robot according to claim 1, wherein the performance indicators include micro-instrument tip dexterity, micro-instrument intra-body cooperation space, and hand-eye coordination.

3. The method for planning initial poses of surgical arms of a laparoscopic surgical robot according to claim 2, wherein the micro-instrument tip dexterity is used to reflect conversion capability of the speed and force of the surgical arm from a joint space to a tip task space, and the micro-instrument tip dexterity is obtained by the following formula:

$$\text{Micro-instrument tip dexterity} = \begin{cases} \dfrac{\displaystyle\int_w \dfrac{\mu_\sigma}{\sigma_\sigma}\,dw}{\displaystyle\int_w dw}, & \sigma_{G\min} \neq 0 \\[4pt] 0, & \sigma_{G\min} = 0 \end{cases}$$

where $\mu_\sigma$ and $\sigma_\sigma$ are a mean value and a standard deviation of singular values of a Jacobian matrix corresponding to a tip posture of the micro-instrument respectively;

$\sigma_{G\min}$ is a minimum singular value of the Jacobian matrix in the surgical region;

w is a minimally invasive surgical region space.

4. The method for planning initial poses of surgical arms of a laparoscopic surgical robot according to claim 3, wherein the greater the value of the micro-instrument tip dexterity is, the more balanced the conversion capability of the speed and force of the surgical arm from the joint space to the tip task space is.

5. The method for planning initial poses of surgical arms of a laparoscopic surgical robot according to claim 2, wherein the micro-instrument intra-body cooperation space is used to reflect a cooperative space for the left and right micro-instruments at the position of the surgical region on the basis of a collision-free working space for the left and right surgical arms at the position of the surgical region, and the micro-instrument intra-body cooperation space is obtained by the following formula:

$$\text{Micro} - \text{instrument intra} - \text{body cooperation space} = \frac{v_{cr} \cap v_{cc}}{v_c} \times 100\%, v_{cr} \in v_c, v_{cc} \in v_c$$

where, $v_c$ is the surgical region position space;

$v_{cr}$ is the cooperative space for the left and right micro-instruments at the position of the surgical region;

$v_{cc}$ is a collision-free working space for the left and right surgical arms at the position of the surgical region.

6. The method for planning initial poses of surgical arms of a laparoscopic surgical robot according to claim 5, wherein the greater the value of the micro-instrument intra-body cooperation space is, the larger the cooperative space for the left and right micro-instruments at the position of the surgical region is on the basis of the collision-free working space for the left and right surgical arms at the position of the surgical region.

7. The method for planning initial poses of surgical arms of a laparoscopic surgical robot according to claim 2, wherein the hand-eye coordination is used to reflect a positional relationship between the left and right micro-instruments and a visual field relationship between the left and right micro-instruments and an endoscope; the positional relationship between the left and right micro-instruments includes an operating angle of the left and right micro-instruments and an elevation angle of the left and right micro-instruments; and the visual field relationship between the left and right micro-instruments and the endoscope includes an azimuth angle of the left and right micro-instruments relative to the direction of visual field.

8. The method for planning initial poses of surgical arms of a laparoscopic surgical robot according to claim 7, wherein the operating angle of the left and right micro-instruments is 60°; the elevation angle of the left and right micro-instruments is 60°; and the azimuth angles of the left and right micro-instruments relative to the direction of visual field are the same.

9. The method for planning initial poses of surgical arms of a laparoscopic surgical robot according to any one of claims 1-8, wherein the obtaining a plurality of initial position schemes for a surgical arm joint on the basis of the position of the minimally invasive incision comprises:

performing a kinematic inverse operation according to the position of the minimally invasive incision and the position of the surgical region to obtain a plurality of combinations of the positions of the surgical arm joint;

performing an inter-arm collision test on each of the surgical arm joint position combinations to obtain a plurality of initial position schemes for the surgical arm joint that do not generate an inter-arm collision.

10. A device for planning initial poses of surgical arms of a laparoscopic surgical robot, comprising:

an acquisition module configured for acquiring positions set for a minimally invasive incision and a surgical region;
a scheme generation module configured for obtaining a plurality of initial position schemes for a surgical arm joint on the basis of the position of the minimally invasive incision and the position of the surgical region; and
[0075] a calculation module configured for calculating performance indicators of each initial position scheme for the surgical arm joint respectively.

Acquiring positions set for a minimally invasive incision and a surgical region ⟩~⟨S1

Obtaining a plurality of initial position schemes for a surgical arm joint on the basis of the position of the minimally invasive incision and the position of the surgical region ⟩~⟨S2

Calculating performance indicators of each initial position scheme for the surgical arm joint according to the position of the surgical region respectively ⟩~⟨S3

Fig. 1

Fig. 2

Performing a kinematic inverse operation according to the position of the minimally invasive incision and the position of the surgical region to obtain a plurality of combinations of the positions of the surgical arm joint

S21

Performing an inter-arm collision test on each of the surgical arm joint position combinations to obtain a plurality of initial position schemes for the surgical arm joint that do not generate an inter-arm collision

S22

Fig. 3

Fig. 4

Acquisition module

Scheme generation module

Calculation module

Fig. 5

## INTERNATIONAL SEARCH REPORT

| International application No. |
|---|
| **PCT/CN2021/093787** |

| A. | CLASSIFICATION OF SUBJECT MATTER |
|---|---|
| | A61B 34/10(2016.01)i; A61B 34/30(2016.01)i |

According to International Patent Classification (IPC) or to both national classification and IPC

| B. | FIELDS SEARCHED |
|---|---|

Minimum documentation searched (classification system followed by classification symbols)

    A61B

Documentation searched other than minimum documentation to the extent that such documents are included in the fields searched

Electronic data base consulted during the international search (name of data base and, where practicable, search terms used)

    CNPAT, CNKI, WPI, EPODOC: 哈尔滨思哲睿智能医疗设备有限公司, 王伟, 机器人, 术前, 摆位, 位置, 初始, 方案, 创口, 切口, 体型, 空间, 机械臂, 微器械, 器械臂, 干涉, 碰撞, 避障, 关节, 坐标, 规划, 灵巧度, 协调, 协作, program+, surgical+, operat+, initial, pose, plan+, position, scheme, area, calculat+, incision, joint, medical+, invasive, optimiz+, robot, assist+, place+

| C. | DOCUMENTS CONSIDERED TO BE RELEVANT |
|---|---|

| Category* | Citation of document, with indication, where appropriate, of the relevant passages | Relevant to claim No. |
|---|---|---|
| X | 王伟 等 (WANG, Wei et al.). "基于协作空间与灵巧度的机器人辅助微创手术术前规划算法 (A Preoperative Planning Algorithm Based on Dexterity and Collaboration Space for the Robot-Assisted Minimally Invasive Surgery)" 机器人 *(Robot)*, Vol. 38, No. 2, 31 March 2016 (2016-03-31), ISSN: 1002-0446, full text, sections 3-4 | 1, 9-10 |
| Y | 王伟 等 (WANG, Wei et al.). "基于协作空间与灵巧度的机器人辅助微创手术术前规划算法 (A Preoperative Planning Algorithm Based on Dexterity and Collaboration Space for the Robot-Assisted Minimally Invasive Surgery)" 机器人 *(Robot)*, Vol. 38, No. 2, 31 March 2016 (2016-03-31), ISSN: 1002-0446, full text, sections 3-4 | 2-8 |
| Y | 梁科 等 (LIANG, Ke et al.). "应用于机器人辅助微创外科手术的术前规划方法 (Preoperative Planning Algorithm for Robot-Assisted Minimally Invasive Surgery)" 天津大学学报 *(自然科学与工程技术版) (Journal of Tianjin University (Science and Technology))*, Vol. 52, No. 9, 30 September 2019 (2019-09-30), ISSN: 0493-2137, full text, sections 3-4 | 2-8 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 October 2021** | **01 November 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2021/093787**

**C.    DOCUMENTS CONSIDERED TO BE RELEVANT**

| Category* | Citation of document, with indication, where appropriate, of the relevant passages | Relevant to claim No. |
|---|---|---|
| A | CN 107030702 A (HUAZHONG UNIVERSITY OF SCIENCE AND TECHNOLOGY) 11 August 2017 (2017-08-11)<br>      entire document | 1-10 |
| A | US 2017049517 A1 (ETHICON ENDO-SURGERY, L.L.C.) 23 February 2017 (2017-02-23)<br>      entire document | 1-10 |
| A | CN 111546347 A (NAVAL UNIVERSITY OF ENGINEERING, PLA.) 18 August 2020 (2020-08-18)<br>      entire document | 1-10 |
| A | FENG, Mei et al. "Pose optimization and port placement for robot-assisted minimally invasive surgery in cholecystectomy"<br>*INTERNATIONAL JOURNAL OF MEDICAL ROBOTICS AND COMPUTER ASSISTED SURGERY*, Vol. 13, No. 4, 31 December 2017 (2017-12-31),<br>ISSN: 1478-5951,<br>      entire document | 1-10 |

Form PCT/ISA/210 (second sheet) (January 2015)

INTERNATIONAL SEARCH REPORT

Information on patent family members

International application No.

**PCT/CN2021/093787**

| Patent document cited in search report | | | Publication date (day/month/year) | Patent family member(s) | | | Publication date (day/month/year) |
|---|---|---|---|---|---|---|---|
| CN | 107030702 | A | 11 August 2017 | None | | | |
| US | 2017049517 | A1 | 23 February 2017 | WO | 2017030848 | A1 | 23 February 2017 |
| | | | | US | 10136949 | B2 | 27 November 2018 |
| CN | 111546347 | A | 18 August 2020 | None | | | |

Form PCT/ISA/210 (patent family annex) (January 2015)